# EUROPÄISCHE PATENTANMELDUNG

(11) **EP 3 400 880 A1**
(43) Veröffentlichungstag der Anmeldung: **14.11.2018**
(21) Anmeldenummer: 18168393.9
(22) Anmeldetag: 05.02.2014
(51) Int. Cl.: A61B 17/02, A61B 17/29

(54) **CHIRURGISCHE RETRAKTIONSVORRICHTUNG**

(30) Priorität: 21.03.2013 DE 102013102902
(62) Teilanmeldung aus: 14703334.4
(71) Anmelder: Aesculap AG, 78532 Tuttlingen (DE)
(72) Erfinder: Weißhaupt, Dieter, 78194 Immendingen (DE); Morales, Pedro, 78532 Tuttlingen-Nendingen (DE); Vogtherr, Robert, 78532 Tuttlingen (DE); Elisch, Andreas, 78713 Schramberg (DE); Kleine, Peter, 63263 Neu Isenburg (DE)
(74) Vertreter: Hoeger, Stellrecht & Partner Patentanwälte mbB

(57) **Zusammenfassung**

Die Erfindung betrifft eine chirurgische Retraktionsvorrichtung, insbesondere zum Spreizen eines durchtrennten Sternums, umfassend eine erste Rückhalteeinrichtung (12) und eine zweite Rückhalteeinrichtung (14), die jeweils einen Spreizarm (16, 18) mit mindestens einem daran gehaltenen Rückhalteelement (26) aufweisen, eine Halteeinrichtung (28) zum Halten der Rückhalteeinrichtungen (12, 14) aneinander sowie eine Antriebseinrichtung (46), mit der der Abstand der Spreizarme (16, 18) voneinander längs einer Retraktionsrichtung (20) zum Überführen in eine Spreizstellung veränderbar ist. Um eine derartige Retraktionsvorrichtung bereitzustellen, mit der bei kompakter Bauform eine einfache Handhabung erzielt werden kann, wird erfindungsgemäß vorgeschlagen, dass die Rückhalteeinrichtungen (12, 14) jeweils einen mit dem jeweiligen Spreizarm (16, 18) verbundenen Haltearm (30, 32) aufweisen, wobei beide Haltearme (30, 32) mit der Antriebseinrichtung (46) koppeln und über diese relativ zueinander und relativ zur Halteeinrichtung (28) längs einer Verschieberichtung (20) verschieblich sind.

## Beschreibung

Die Erfindung betrifft eine chirurgische Retraktionsvorrichtung, insbesondere zum Spreizen eines durchtrennten Sternums, umfassend eine erste Rückhalteeinrichtung und eine zweite Rückhalteeinrichtung, die jeweils einen Spreizarm mit mindestens einem daran gehaltenen Rückhalteelement aufweisen, eine Halteeinrichtung zum Halten der Rückhalteeinrichtungen aneinander sowie eine Antriebseinrichtung, mit der der Abstand der Spreizarme voneinander längs einer Retraktionsrichtung zum Überführen in eine Spreizstellung veränderbar ist.

Eine derartige Retraktionsvorrichtung wird insbesondere bei Operationen am Brustkorb verwendet, um einen Zugang zum Herzen bereitzustellen. Dabei wird das Sternum in Längsrichtung mittig durchtrennt und an jeder Sternumseite ein Spreizarm mit dem jeweiligen mindestens einen Rückhalteelement angelegt. Durch Betätigen der Antriebseinrichtung werden die Spreizarme längs der Retraktionsrichtung von einer ungespreizten Stellung in eine Spreizstellung überführt und das von den Rückhalteelementen zurückgehaltene Sternum aufgespreizt. Üblicherweise definieren die Spreizarme dabei eine Spreizebene, in der die Aufspreizung erfolgt.

Bei bekannten Retraktionsvorrichtungen ist ein Spreizarm relativ zum anderen Spreizarm beweglich, an dem die Halteeinrichtung starr festgelegt ist. Der bewegliche Spreizarm ist zu diesem Zweck üblicherweise mit einem Haltearm verbunden, der an der Halteeinrichtung gehalten ist. Damit die Spreizarme in der Spreizstellung einen für den operativen Eingriff hinreichenden Abstand voneinander aufweisen können, ist es bei den bekannten Retraktionsvorrichtungen erforderlich, dass der Haltearm der beweglichen Rückhalteeinrichtung seitlich nicht unbeträchtlich über die andere Rückhalteeinrichtung hinaussteht. Dies kann vom Operateur als störend empfunden werden. Ein solcher Haltearm kann sich insbesondere auch dann als nachteilig erweisen, wenn eine Sternumhälfte im gespreizten Zustand aus der Spreizebene angehoben wird und dabei mit dem Haltearm kollidieren kann.

Aufgabe der vorliegenden Erfindung ist es, eine gattungsgemäße Retraktionsvorrichtung bereitzustellen, mit der bei kompakter Bauform eine einfache Handhabung erzielt werden kann.

Diese Aufgabe wird bei einer Retraktionsvorrichtung der eingangs genannten Art erfindungsgemäß dadurch gelöst, dass die Rückhalteeinrichtungen jeweils einen mit dem jeweiligen Spreizarm verbundenen Haltearm aufweisen, wobei beide Haltearme mit der Antriebseinrichtung koppeln und über diese relativ zueinander und relativ zur Halteeinrichtung längs einer Verschieberichtung verschieblich sind.

Bei der erfindungsgemäßen Retraktionsvorrichtung umfassen beide Rückhalteeinrichtungen einen Haltearm, wobei die Haltearme relativ zueinander und relativ zur Halteeinrichtung verschieblich sind. Durch Einsatz von nur einer Antriebseinrichtung können die Haltearme und damit die Rückhalteeinrichtungen angetrieben und relativ zueinander längs einer Verschieberichtung verschoben werden. Dadurch kann insbesondere dann, wenn die Halteeinrichtung und die Antriebseinrichtung, bezogen auf die Retraktionsrichtung, zwischen den Spreizarmen angeordnet sind, eine besonders kompakte Bauform der Retraktionsvorrichtung erzielt werden. Dies gibt insbesondere die Möglichkeit, die Rückhalteeinrichtungen gegengleich zum Spreizen in voneinander weg weisenden Richtungen zu verschieben. Anders als bei herkömmlichen Retraktionsvorrichtungen legt jede Rückhalteeinrichtung einen Teil des Weges relativ zur Halteeinrichtung zurück, der erforderlich ist, um die Spreizarme in die Spreizstellung zu überführen. Dies begünstigt die kompaktere Bauform der Rückhaltevorrichtung. Darüber hinaus zeigt sich in der Praxis, dass durch Antreiben beider Haltearme die Antriebseinrichtung einem geringeren Verschleiß unterliegt als bei gattungsgemäßen Retraktionsvorrichtungen, bei denen nur eine der Rückhalteeinrichtungen angetrieben wird.

Günstig ist es, wenn die Verschieberichtung parallel zur Retraktionsrichtung ausgerichtet ist. Der Retraktionsvorrichtung kann dadurch eine konstruktiv einfachere Ausgestaltung verliehen werden.

Von Vorteil ist es, wenn die Halteeinrichtung und/oder die Antriebseinrichtung, bezogen auf die Retraktionsrichtung jeweils zumindest teilweise, in der Mitte oder im Wesentlichen in der Mitte zwischen den Spreizarmen angeordnet ist, um eine kompakte Bauform der Retraktionsvorrichtung zu erzielen. Beispielsweise sind die Halteeinrichtung und/oder die Antriebseinrichtung um eine Mittelebene der Retraktionsvorrichtung zentriert angeordnet, welche Mittelebene senkrecht zu einer von den Spreizarmen definierten Spreizebene ausgerichtet ist. Beide Rückhalteeinrichtungen können sich ausgehend von der Mittelebene unter Antrieb durch die Antriebseinrichtung gegenläufig bewegen, so dass der Retraktionsvorrichtung eine geringe Baubreite verliehen werden kann.

Von besonderem Vorteil ist es, wenn die Halteeinrichtung und/oder die Antriebseinrichtung unabhängig vom Abstand der Spreizarme voneinander in der Mitte oder im Wesentlichen in der Mitte zwischen den Spreizarmen angeordnet ist. Dadurch ist die Möglichkeit gegeben, dass die Halteeinrichtung und/oder die Antriebseinrichtung ortsfest bezüglich des Patientenkörpers verbleiben kann, wohingegen sich die Rückhalteeinrichtungen bei gleichem Verschiebeweg relativ zum Patientenkörper in die Spreizstellung überführen lassen.

Vorzugsweise ist die Halteeinrichtung und/oder die Antriebseinrichtung in oder im Wesentlichen in einer von den Spreizarmen definierten Spreizebene angeordnet. Dadurch kann der Retraktionsvorrichtung eine flache Bauform verliehen werden, was vom Operateur als weniger störend empfunden wird.

Vorzugsweise sind die Haltearme bei Betätigung der Antriebseinrichtung um dieselbe Wegstrecke relativ zur Halteeinrichtung verschiebbar. Dies gibt insbesondere die Möglichkeit, die Rückhalteeinrichtungen symmetrisch zueinander bezüglich der Halteeinrichtung zu verschieben. Die Halteeinrichtung kann dabei unabhängig vom Abstand der Spreizarme voneinander in der Mitte zwischen den Rückhalteelementen angeordnet sein.

Für eine flache und kompakte Bauform der Retraktionsvorrichtung ist es von Vorteil, wenn die Haltearme schienenförmig ausgestaltet sind.

Günstig ist es, wenn die Halteeinrichtung ein Gehäuse umfasst oder ausbildet, das die Haltearme zumindest teilweise umgreift, das Gehäuse kann die Haltearme dadurch zusammenhalten. Mindestens ein Antriebselement der Antriebseinrichtung kann im Gehäuse angeordnet sein, und an den Haltearmen angeordnete Antriebselemente oder Antriebsbahnen, darauf wird nachfolgend noch eingegangen, können zumindest teilweise im Gehäuse angeordnet sein. Die Antriebsbahnen und/oder das Antriebselement können dadurch einem unmittelbaren Zugriff zumindest teilweise entzogen werden. Dies verringert beispielsweise die Wahrscheinlichkeit, dass sich chirurgisches Nahtmaterial an dem Antriebselement oder den Antriebsbahnen verfängt und diese blockiert.

Vorteilhafterweise umgreift das Gehäuse die Haltearme vollständig oder im Wesentlichen vollständig. Das Gehäuse kann in einer Ebene quer zur Verschieberichtung günstigerweise in sich geschlossen sein.

Von Vorteil ist es, wenn das Gehäuse als Flachgehäuse ausgestaltet ist. Das Flachgehäuse kann Breit-und Schmalseiten aufweisen, wobei im bestimmungsgemäßen Gebrauch der Retraktionsvorrichtung vorzugsweise eine Breitseite dem Patientenkörper zugewandt ist. Bei Anwendung der Retraktionsvorrichtung zum Spreizen eines Sternums lässt sich die Retraktionsvorrichtung dadurch beispielsweise besser auf den Brustkorb auflegen.

Die Haltearme können von einander gegenüberliegenden Seiten in das Gehäuse eingreifen und/oder dieses durchgreifen. Beim Spreizen der Rückhalteeinrichtungen relativ zueinander können freie Enden der Haltearme in Richtung auf das Gehäuse verschoben werden und eine mit dem jeweiligen Spreizarm verbundene Seite eines Haltearmes vom Gehäuse weggeschoben werden.

Werden umgekehrt die Spreizarme aneinander angenähert, kann die jeweilige mit einem Spreizarm verbundene Seite eines Haltearms in Richtung auf das Gehäuse verschoben und ein freies Ende des Haltearms vom Gehäuse weg verschoben werden.

Von Vorteil ist es, wenn die Halteeinrichtung eine Führung für zumindest einen der Haltearme bildet und bevorzugt für beide Haltearme. Dadurch kann eine zuverlässige Funktion der Retraktionsvorrichtung sichergestellt werden.

Ergänzend oder alternativ ist es zur Erzielung desselben Vorteils günstig, wenn die Haltearme aneinander anliegen und sich beim Verschieben relativ zueinander gegenseitig führen.

Beispielsweise kann vorgesehen sein, dass die Haltearme Breitseiten und Schmalseiten aufweisen und dass die Haltearme über die jeweiligen Breitseiten flächig aneinander anliegen, wobei sie sich gegenseitig führen können.

Die Haltearme sind vorzugsweise in oder im Wesentlichen in der Spreizebene angeordnet, wodurch die Retraktionsvorrichtung eine flache Bauform aufweisen kann.

Bevorzugt ist die Antriebseinrichtung an der Halteeinrichtung angeordnet oder wird von dieser umfasst. Beispielsweise ist die Antriebseinrichtung zumindest teilweise in die Halteeinrichtung integriert, insbesondere mindestens ein Antriebselement der Antriebseinrichtung in einem Gehäuse der Halteeinrichtung angeordnet.

Bei einer konstruktiv einfachen Retraktionsvorrichtung, deren zuverlässige Funktion sichergestellt werden kann, ist es günstig, wenn die Antriebseinrichtung mindestens ein Antriebselement umfasst und wenn die Haltearme jeweils eine sich in Verschieberichtung erstreckende Antriebsbahn umfassen, wobei das mindestens eine Antriebselement mit der jeweiligen Antriebsbahn zusammenwirkt. Durch Einwirken auf das mindestens eine Antriebselement kann die jeweilige Antriebsbahn eines Haltearmes angetrieben und dadurch der Haltearm verschoben werden.

Das mindestens eine Antriebselement kann in oder im Wesentlichen in der Mitte zwischen den Spreizarmen angeordnet sein, bezogen auf die Retraktionsrichtung und vorzugsweise unabhängig vom Abstand der Spreizarme voneinander.

Das mindestens eine Antriebselement und/oder die Antriebsbahnen können verschleißmindernden Herstellungs- oder Bearbeitungsverfahren unterzogen sein und/oder verschleißarme Materialien umfassen oder aus solchen Materialien gefertigt sein. Beispielsweise können Oberflächen- und/oder Wärmebehandlungen wie etwa Nitrieren, Randschichtenhärten, Borieren, Einsatzhärten oder dergleichen an dem mindestens einen Antriebselement und/oder den Antriebsbahnen durchgeführt sein. Ebenfalls ist es möglich, einen gezielten Härteunterschied zwischen den zusammenwirkenden mindestens einen Antriebselement und den Antriebsbahnen bereitzustellen, um den Verschleiß zu optimieren.

Der Verschleiß lässt sich ferner durch den Einsatz geeigneter Materialien verringern, beispielsweise durch eine verschleißgünstige Materialkombination aus Keramik oder Kunststoff einerseits und Metall andererseits.

Entsprechende Verfahren und/oder entsprechende Materialien können bei allen relativ zueinander bewegbaren Teilen der Retraktionsvorrichtung zum Einsatz kommen. Beispielsweise können die Haltearme einerseits und ein Gehäuse der Halteeinrichtung andererseits hinsichtlich einer Verschleißminderung optimiert sein. Denkbar ist auch, dass das nachfolgend noch erwähnte Betätigungselement der Antriebseinrichtung und insbesondere ein Gehäuse der Halteeinrichtung hinsichtlich einer Verschleißminderung aneinander angepasst sind.

Das mindestens eine Antriebselement und die Antriebsbahnen können einen Lineartrieb bilden, so dass die Haltearme auf konstruktiv einfache Weise angetrieben werden können.

Günstigerweise sind die Antriebsbahnen identisch ausgestaltet.

Als vorteilhaft erweist es sich, wenn die Antriebsbahn als am jeweiligen Haltearm angeordnete Zahnreihe ausgestaltet ist und wenn das mindestens eine Antriebselement als Zapfenrad oder als Zahnrad ausgestaltet ist, das mit der Zahnreihe kämmt. Dies erlaubt es, die Haltearme über eine Verzahnung mit dem mindestens einen Antriebselement anzutreiben, zum Beispiel eine Triebstockverzahnung.

Bei einer andersartigen vorteilhaften Ausführungsform der erfindungsgemäßen Retraktionsvorrichtung kann vorgesehen sein, dass als Antriebsbahn eine Rollenbahn zum Einsatz kommt und dass das mindestens eine Antriebselement als auf der Rollenbahn abrollende Rolle ausgestaltet ist.

Günstigerweise ist das mindestens eine Antriebselement an der Halteeinrichtung drehbar gelagert um eine Drehachse senkrecht zur Verschieberichtung. Durch Drehen des Antriebselementes, insbesondere eines Zapfenrades oder eines Zahnrades, kann dadurch der jeweilige Haltearm angetrieben werden.

Von Vorteil ist es, wenn die Antriebseinrichtung zum Einwirken auf das mindestens eine Antriebselement mindestens ein handbetätigbares Betätigungselement umfasst, vorzugsweise in Gestalt einer Kurbel oder eines Drehgriffes. Durch Drehen der Kurbel oder des Drehgriffes kann das mindestens eine Antriebselement insbesondere um die vorstehend erwähnte Drehachse gedreht werden und kämmt dabei vorzugsweise mit der vorstehend erwähnten Zahnreihe.

Bevorzugt ist das Betätigungselement entfernbar. Nehmen die Spreizarme eine gewünschte Spreizstellung ein, kann das Betätigungselement entfernt werden, um dem Operateur einen noch besseren Zugang zum Operationsfeld zu ermöglichen. Das mindestens eine Betätigungselement ist vorzugsweise werkzeuglos entfernbar.

Vorteilhafterweise umfasst die Retraktionsvorrichtung eine Fixiereinrichtung, mit der die Rückhalteeinrichtungen in einer Spreizstellung fixierbar sind, speziell nach Entfernen des Betätigungselementes.

Bei einer vorteilhaften Ausführungsform erweist es sich als günstig, wenn mindestens ein Haltearm ein längs der Verschieberichtung ausgerichtetes Langloch umfasst, an dessen Rand die Antriebsbahn angeordnet ist. Dadurch ist die Wahrscheinlichkeit, dass sich Fremdkörper wie zum Beispiel chirurgisches Nahtmaterial an der Zahnreihe verfangen, verringerbar.

Es kann vorgesehen sein, dass die Antriebsbahnen an einander abgewandten Seiten der Haltearme angeordnet sind. Bei einer derartigen Ausführungsform können insbesondere zwei Antriebselemente vorgesehen sein, deren jeweils eines mit einer Antriebsbahn zusammenwirkt.

In der Praxis erweist es sich als vorteilhaft, wenn die Haltearme Breitseiten und Schmalseiten aufweisen und wenn die Antriebsbahnen an den Schmalseiten angeordnet sind. In diesem Fall ist es möglich, dass die Antriebsbahnen an einander zugewandten oder an einander abgewandten Schmalseiten angeordnet sind.

Die Antriebsbahnen können in einer gemeinsamen Ebene oder in parallelen Ebenen angeordnet sein, bevorzugt parallel zur Spreizebene oder in dieser.

Als günstig erweist es sich, wenn die Antriebseinrichtung ein Antriebselement umfasst, das mit den Antriebsbahnen beider Haltearme zusammenwirkt. Beispielsweise ist ein Antriebselement in Gestalt eines Zapfenrades oder Zahnrades vorgesehen, das mit zwei als Zahnreihen ausgestalteten Antriebsbahnen gleichzeitig kämmt. Durch eine Drehung des Antriebselementes können die Rückhalteelemente dadurch in einen Abstand voneinander gebracht werden, der doppelt so groß ist wie der Verschiebeweg jedes Haltearmes.

Günstigerweise kann zur Erzielung einer kompakten Bauform vorgesehen sein, dass die Antriebsbahnen der Haltearme einander zugewandt sind und dass das Antriebselement in einen Raum zwischen den Antriebsbahnen eingreift. Die Antriebsbahnen sind beispielsweise an Schmalseiten der Haltearme angeordnet, die einander zugewandt sind. Es kann auch vorgesehen sein, dass die Haltearme über Breitseiten aneinander anliegen und jeweils ein längs der Verschieberichtung ausgestrecktes Langloch umfassen, an dessen Rand die Antriebsbahnen angeordnet sind.

Bei einer andersartigen vorteilhaften Ausführungsform der erfindungsgemäßen Retraktionsvorrichtung ist es günstig, wenn die Antriebseinrichtung zwei Antriebselemente umfasst, wobei jedes Antriebselement mit einer Antriebsbahn eines Haltearmes zusammenwirkt. Die Antriebselemente koppeln mit jeweils einer Antriebsbahn, die beispielsweise an einander abgewandten Seiten der Haltearme angeordnet sind.

Als vorteilhaft erweist es sich, wenn die Antriebselemente gemeinsam antreibbar sind. Vorzugsweise umfasst die Antriebseinrichtung ein mit dem Betätigungselement gekoppeltes weiteres Antriebselement, das mit den beiden Antriebselementen kämmt. Dies gibt insbesondere die Möglichkeit, ein Getriebe bei der Antriebseinrichtung vorzusehen. Durch das Betätigungselement kann ein Antriebselement angetrieben werden, welches mit den beiden mit den Antriebsbahnen zusammenwirkenden Antriebselementen koppelt. Dies gibt die Möglichkeit, eine gewünschte Übersetzung oder Untersetzung zwischen den koppelnden Antriebselementen vorzusehen. Dies erlaubt es auf konstruktiv einfache Weise, die Drehzahl der Antriebselemente und damit den Verstellweg der Haltearme der Anforderung bedarfsgerecht einzustellen.

Insgesamt ist es günstig, wenn die Antriebseinrichtung ein Getriebe umfasst oder ausbildet, mit der eine Drehzahl des als Drehkörper, insbesondere als Zahnrad oder Zapfenrad, ausgestalteten mindestens einen Antriebselementes veränderbar ist.

Bevorzugt ist das Getriebe in einem von der Halteeinrichtung umfassten oder gebildeten Gehäuse aufgenommen. Das Getriebe kann dadurch vorteilhafterweise dem unmittelbaren Zugriff entzogen. Die Wahrscheinlichkeit, dass Fremdkörper wie zum Beispiel chirurgisches Nahtmaterial in das Getriebe eingezogen werden, wird dadurch verringert.

Als günstig erweist es sich, wenn zumindest einer der Haltearme eine Führung an der jeweils anderen Rückhalteeinrichtung durchgreift. Beispielsweise ist an einem Spreizarm einer Rückhalteeinrichtung eine Führung an einem Rand einer Durchgangsöffnung für einen Haltearm der anderen Rückhalteeinrichtung vorgesehen.

Zur Erzielung einer konstruktiv einfachen Ausgestaltung kann vorgesehen sein, dass jeder Haltearm einstückig mit dem jeweiligen Spreizarm verbunden ist.

Die nachfolgende Beschreibung bevorzugter Ausführungsformen der Erfindung dient im Zusammenhang mit der Zeichnung der näheren Erläuterung der Erfindung. Es zeigen:
- Figur 1:: eine perspektivische Darstellung einer ersten bevorzugten Ausführungsform einer erfindungsgemäßen Retraktionsvorrichtung;

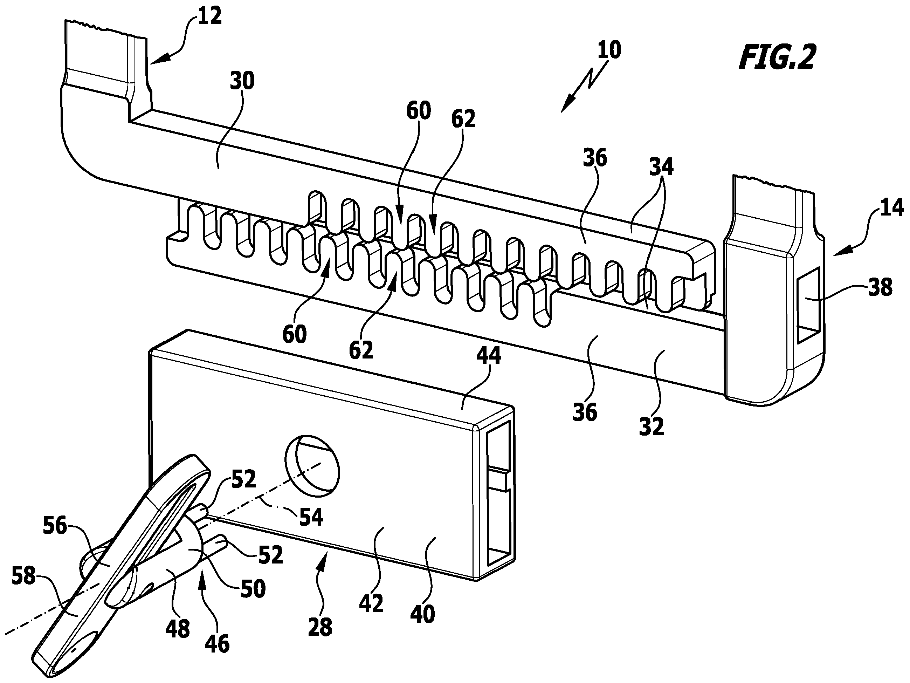

- Figur 2:: eine Teilansicht der Retraktionsvorrichtung aus Figur 1 in einer Explosionsdarstellung;
- Figur 3:: eine Teildarstellung der Retraktionsvorrichtung aus Figur 1 in einer Draufsicht;
- Figur 4:: eine perspektivische Darstellung einer zweiten bevorzugten Ausführungsform einer erfindungsgemäßen Retraktionsvorrichtung;
- Figur 5:: eine Teilansicht der Retraktionsvorrichtung aus Figur 4 in einer Explosionsdarstellung;
- Figur 6:: eine Teildarstellung der Retraktionsvorrichtung aus Figur 4 in einer Draufsicht;
- Figur 7:: eine perspektivische Darstellung einer dritten bevorzugten Ausführungsform einer erfindungsgemäßen Retraktionsvorrichtung;
- Figur 8:: eine Teilansicht der Retraktionsvorrichtung aus Figur 7 in einer Explosionsdarstellung; und
- Figur 9:: eine Teildarstellung der Retraktionsvorrichtung aus Figur 7 in einer Draufsicht.

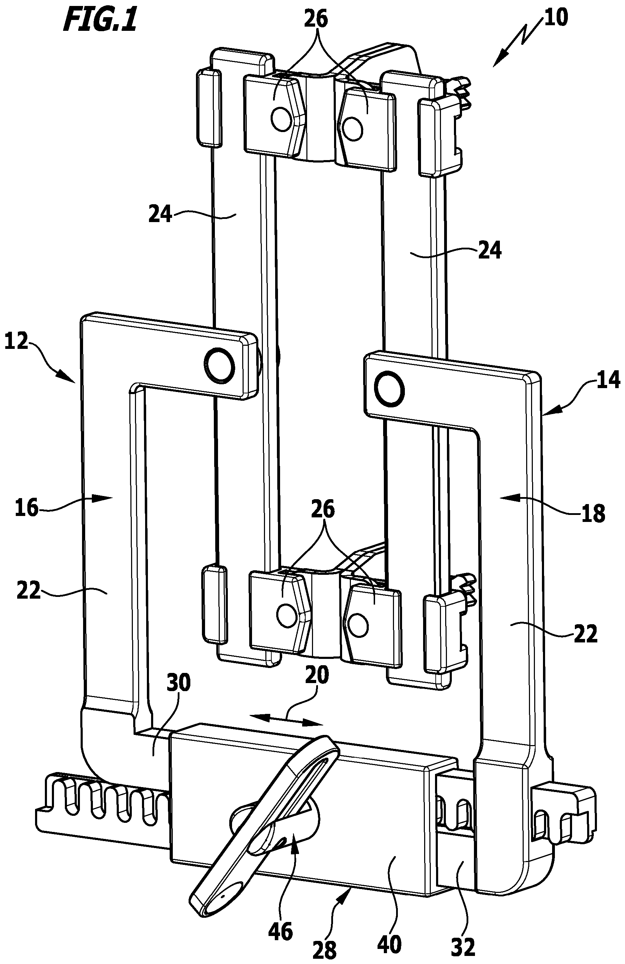

Figur 1 zeigt in perspektivischer Darstellung eine insgesamt mit dem Bezugszeichen 10 belegte erste bevorzugte Ausführungsform einer erfindungsgemäßen chirurgischen Retraktionsvorrichtung. Die Retraktionsvorrichtung 10 ist insbesondere zum Spreizen eines in der Zeichnung nicht dargestellten Sternums vorgesehen, um einem Operateur beispielsweise bei einer Herzoperation einen Zugang in den geöffneten Brustkorb zu ermöglichen.

Die Retraktionsvorrichtung 10 umfasst zwei Rückhalteeinrichtungen 12 und 14. Die Rückhalteeinrichtungen 12, 14 umfassen Spreizarme 16 bzw. 18, die in einen variablen Abstand zueinander gebracht werden können, indem sie auf nachfolgend noch erläuterte Weise längs einer Retraktionsrichtung 20 relativ zueinander verschoben werden. Die Retraktionsrichtung 20 ist daher auch eine Verschieberichtung 20 der Retraktionsvorrichtung.

Die genaue Ausgestaltung der Spreizarme 16, 18 im Einzelnen ist für das Verständnis der vorliegenden Erfindung nicht von Bedeutung. Wesentlich ist lediglich, dass die Spreizarme 16, 18 mindestens jeweils ein Rückhalteelement umfassen und durch Verschieben relativ zueinander in einen variablen Abstand gebracht werden können. Dabei können sie längs der Retraktionsrichtung 20 in eine Spreizstellung (nicht gezeigt) überführt werden. Die nicht-vorveröffentlichte Patentanmeldung DE 10 2012 100 284.3 derselben Anmelderin umfasst eine Mehrzahl weiterer Ausgestaltungen von Spreizarmen, die beispielsweise bei der Retraktionsvorrichtung 10 anstelle der Spreizarme 16, 18 zum Einsatz kommen könnten. In Bezug auf derartige Ausgestaltungen wird Bezug genommen auf die Patentanmeldung DE 10 2012 100 284.3, deren Offenbarungsgehalt vollinhaltlich in die vorliegende Anmeldung mit aufgenommen wird.

Jeder Spreizarm 16, 18 umfasst einen ersten Abschnitt 22, der L-förmig ausgestaltet ist, wobei die Abwinklung in Richtung des jeweils anderen Spreizarmes 18, 16 weist. Weiter umfasst jeder Spreizarm 16, 18 einen zweiten Abschnitt 24, der endseitig am ersten Abschnitt 22 um eine Drehachse verschwenkbar gelagert ist. Die Drehachse ist senkrecht zu einer von den Spreizarmen 16, 18 definierten Spreizebene ausgerichtet, in der das Sternum unter Einsatz der Retraktionsvorrichtung 10 gespreizt wird. Die zweiten Abschnitte 24 sind geradlinig und längserstreckt ausgebildet und ungefähr an ihrer Mitte an den ersten Abschnitten 22 verschwenkbar gelagert. Im Bereich von Endabschnitten ist an jedem zweiten Abschnitt 24 ein Rückhalteelement 26 gehalten. Die Rückhalteelemente 26 umfassen jeweils einen Haltewinkel, der an das Sternum angelegt werden kann. Die Rückhalteelemente 26 der Spreizarme 16, 18 werden dabei so an beide Sternumhälften angelegt, dass diese unter Bewegung der Spreizarme 16, 18 gespreizt werden können. Die Rückhalteelemente 26 können positionsveränderlich an den zweiten Abschnitten 24 gehalten sein, beispielsweise schwenkbar relativ zur Spreizebene an diesen gelagert sein oder längs der zweiten Abschnitte 24 verschiebbar sein.

Die Retraktionsvorrichtung 10 umfasst eine Halteeinrichtung 28, um die Rückhalteeinrichtungen 12, 14 aneinander zu haltern. Zu diesem Zweck weisen die Rückhalteeinrichtungen 12, 14 Haltearme 30 bzw. 32 auf. Beide Haltearme 30, 32 sind geradlinig und längserstreckt ausgestaltet in Form von Schienen, die Schmalseiten 34 und Breitseiten 36 aufweisen. Der Haltearm 30 ist am Spreizarm 16 mit dessen erstem Abschnitt 22 bevorzugt einstückig verbunden, und zwar an dem dem zweiten Abschnitt 24 gegenüberliegenden Ende des ersten Abschnittes 22. Der Haltearm 30 ist relativ zum ersten Abschnitt 22 abgewinkelt, vorzugsweise um 90°, wobei er in Richtung des Spreizarms 18 weist.

In entsprechender Weise ist am ersten Abschnitt 22 des Spreizarms 18 der Haltearm 32 bevorzugt einstückig festgelegt. Der Haltearm 32 ist relativ zum ersten Abschnitt 22 abgewinkelt, vorzugsweise um 90°, wobei er in Richtung des Spreizarms 16 weist. Die Verbindung am ersten Abschnitt 22 erfolgt an dessen dem zweiten Abschnitt 24 gegenüberliegenden Ende.

Der erste Abschnitt 22 des Spreizarms 18 weist eine Durchgangsöffnung 38 auf. Der Haltearm 30 kann die Durchgangsöffnung 38 spielfrei durchgreifen oder in diese eingreifen und dadurch am Spreizarm 18 geführt werden. Nimmt die Retraktionsvorrichtung 10 eine ungespreizte Stellung ein (Figur 1), bei der die Rückhalteelemente 26 beider Spreizarme 16, 18 aneinander anliegen, durchgreift der Haltearm 30 die Durchgangsöffnung 38. Selbst bei etwas gespreizter Retraktionsvorrichtung 10 (nicht gezeigt) kann der Haltearm 30 vom Spreizarm 18 noch geführt werden.

Die Haltearme 30, 32 sind in einer Ebene fluchtend miteinander ausgerichtet und so angeordnet, dass Schmalseiten 34 einander zugewandt sind.

Die Halteeinrichtung 28 umfasst ein Gehäuse 40, das als Flachgehäuse ausgestaltet ist. Das Gehäuse 40 weist dementsprechend zwei Breitseiten 42 und zwei Schmalseiten 44 auf. Die Breitseiten 42 und Schmalseiten 44 bilden einen Mantel, der beide Haltearme 30, 32 umgreift und insbesondere umgibt. Das Gehäuse 40 ist mit anderen Worten in Umfangsrichtung der Haltearme 30, 32 in sich geschlossen. Die Haltearme 30, 32 greifen von einander gegenüberliegenden Seiten in das Gehäuse 40 ein und können dieses durchgreifen. Je nachdem, wie weit die Retraktionsvorrichtung 10 gespreizt ist und welche Spreizstellung die Spreizarme 16, 18 einnehmen, ragen die Haltearme 30, 32 mit ihren freien Enden noch aus dem Gehäuse 40 vor. Ist die Retraktionsvorrichtung 10 weit gespreizt, können die freien Enden der Haltearme 30, 32 auch im Gehäuse 40 angeordnet sein.

Das Gehäuse 40 dient über die Halterung der Rückhalteeinrichtungen 12, 14 hinaus auch deren Führung, wenn die Haltearme 30, 32 relativ zum Gehäuse 40 und relativ zueinander längs der Retraktionsrichtung 20 verschoben werden.

Über eine der Breitseiten 42, die in Anwendung der Retraktionsvorrichtung 10 dem Oberkörper des Patienten zugewandt ist, kann die Retraktionsvorrichtung 10 flächig auf dessen Brustkorb anliegen.

An der Halteeinrichtung 28 ist ferner eine Antriebseinrichtung 46 der Retraktionsvorrichtung 10 angeordnet. Die Antriebseinrichtung 46 weist ein Antriebselement 48 auf, das vorliegend ausgestaltet ist als Zapfenrad 50. Das Zapfenrad 50 ist zylindrisch ausgestaltet, wobei es an einer im Gehäuse 40 angeordneten Stirnseite zwei Zapfen 52 umfasst. Die Zapfen 52 liegen einander bezüglich einer vom Zapfenrad 50 definierten Drehachse 54 diametral gegenüber. Die Drehachse 54 ist senkrecht zur Spreizebene ausgerichtet. An dem den Zapfen 52 gegenüberliegenden Ende ist am Zapfenrad 50 ein Betätigungselement 56 der Antriebselement 46 gehalten. Das Betätigungselement 56 ist handbetätigbar und vorliegend ausgestaltet als Kurbel 58. Das Zapfenrad 50 und die damit verbundene Kurbel 58 sind am Gehäuse 40 drehbar um die Drehachse 54 gelagert.

Es kann vorgesehen sein, dass die Kurbel 58 vom Zapfenrad 50 oder gemeinsam mit diesem vom Gehäuse 40 entfernbar ist, um in Anwendung der Retraktionsvorrichtung 10 einem Operateur nach dem Spreizen des Sternums einen noch besseren Zugang zum Operationsfeld zu ermöglichen.

Die Antriebseinrichtung 46 umfasst ferner zwei Zahnreihen 60. Die Zahnreihen 60 sind an den Haltearmen 30, 32 angeordnet, und zwar jeweils an der dem jeweils anderen Haltearm 30, 32 zugewandten Schmalseite 34. Jede Zahnreihe 60 umfasst eine Mehrzahl von Zähnen, von denen vorliegend 11 Zähne vorgesehen sind. Die Zähne werden dadurch gebildet, dass an den Schmalseiten 34 an den Haltearmen 30, 32 Ausnehmungen gebildet sind, bei denen die übriggebliebenen Zähne ausgenommen wurden. Die Zähne sind jeweils gleichmäßig voneinander beabstandet, und sie erstrecken sich von den freien Enden der Haltearme 30, 32 über ungefähr 60 % von deren Länge.

Das Zapfenrad 50 und die Zahnreihen 60 bilden Triebstockverzahnungen, die als Lineartriebe ausgestaltet sind.

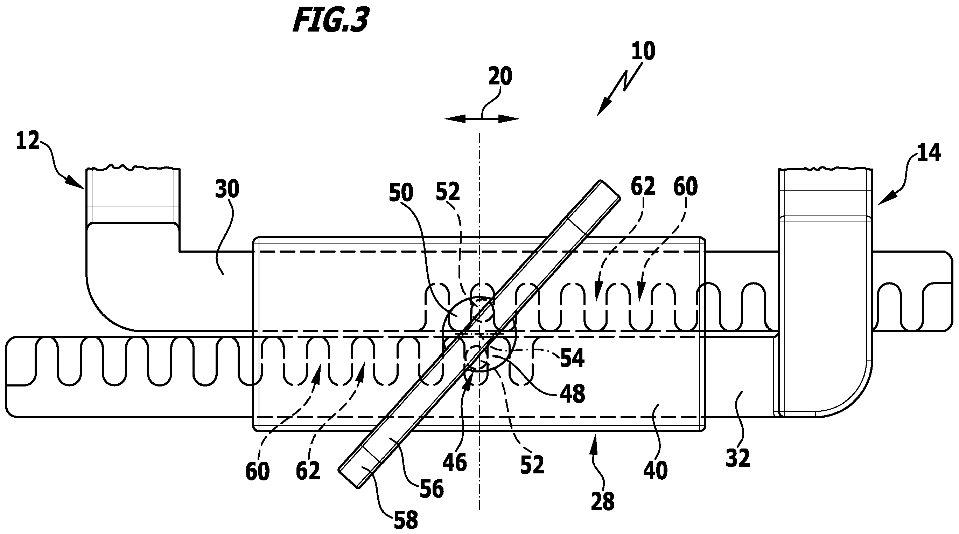

Beide Zahnreihen 60 können gleichzeitig mit dem Zapfenrad 50 koppeln, so dass beide Rückhalteeinrichtungen 12, 14 unter Einsatz nur einer Kurbel 58 angetrieben und relativ zueinander verschoben werden können. Wie insbesondere aus Figur 3 hervorgeht, greifen Zapfen 52 in die Zwischenräume zwischen den Zähnen beider Zahnreihen 60 ein. Durch Drehen der Kurbel 58 und damit des Zapfenrades 50 um die Drehachse 54 können die Zapfen 52 in wechselnden Eingriffen mit den Zähnen an den Haltearmen 30 und 32 gelangen und dadurch beide Haltearme 30, 32 längs der Retraktionsrichtung 20 relativ zueinander verschieben. Da beide Haltearme 30, 32 angetrieben werden können, werden die Zahnreihen 60 auch als Antriebsbahnen 62 der Antriebseinrichtung 46 bezeichnet.

Beim Drehen der Kurbel 58 werden beide Haltearme 30, 32 relativ zueinander und relativ zum Gehäuse 40 längs der Retraktionsrichtung 20 gegengleich verschoben. Weil das Zapfenrad 50 mit beiden Zahnreihen 60 kämmt, werden bei einer Umdrehung die Haltearme 30, 32 relativ zueinander um eine Strecke verschoben, die doppelt so groß ist, als würde nur ein Haltearm 30, 32 mit einer Zahnreihe 60 angetrieben. Dies erlaubt es, der Retraktionsvorrichtung 10 eine kompakte Bauform zu verleihen. Wie insbesondere aus Figur 1 hervorgeht, können die Haltearme 30, 32 so kurz bemessen sein, dass sie bei ungespreizter Retraktionsvorrichtung 10 nicht oder nur geringfügig über den jeweils anderen Spreizarm 16, 18 hinausragen.

Zusätzlich ist es von Vorteil, dass die Halteeinrichtung 28, das Zapfenrad 50 und die Kurbel 58, bezogen auf die Retraktionsrichtung 20, in einer Mittelebene senkrecht zur Spreizebene zwischen den Spreizarmen 16, 18 angeordnet sind. Insbesondere sind die Halteeinrichtung 28, das Zapfenrad 50 und die Kurbel 58 in der Mitte zwischen den ersten Abschnitten 22 angeordnet. Werden die Spreizarme 16, 18 gespreizt, bleiben dadurch die Haltearme 30, 32 über das Gehäuse 40 weiterhin aneinander gehalten.

Bei einer nur geringen Spreizung ragen die Haltearme 30, 32 bereits nicht mehr über den jeweils anderen Spreizarm 16, 18 hervor. Diese Konstruktion erweist sich über die kompakte Bauform hinaus als besonders benutzerfreundlich, da sie vom Operateur als weniger störend empfunden wird. Der Zugang zum Operationsfeld wird durch die Retraktionsvorrichtung 10 in geringerem Umfang versperrt, als dies bei gattungsgemäßen Retraktionsvorrichtungen der Fall ist.

Die Anordnung der Zahnreihen 60 zumindest teilweise innerhalb des Gehäuses 40 verringert die Wahrscheinlichkeit, dass Fremdkörper wie insbesondere chirurgisches Nahtmaterial sich an den Zahnreihen 60 verfangen. Dies erlaubt eine zuverlässigere Funktion der Retraktionsvorrichtung 10.

Darüber hinaus weist die Retraktionsvorrichtung 10 einen nur geringen Verschleiß zwischen aneinander reibenden Bauteilen auf. Das Gehäuse 40 einerseits und das Zapfenrad 50 andererseits sind verschleißmindernd gefertigt oder behandelt, beispielsweise durch Oberflächen- oder Wärmebehandlung wie etwa Nitrieren, Randschichtenhärten, Borieren, Einsatzhärten oder dergleichen. Ein gezielter Härteunterschied von beispielsweise mindestens ungefähr 25 % zwischen den beiden Reibpartnern ist möglich. Zur Verminderung der Reibung können die Materialien aneinander angepasst sein, beispielsweise eine Materialkombination aus Keramik und Metall oder aus Kunststoff und Metall.

Ferner sind Gleitlager und/oder Kugellager möglich, um die Reibung zwischen dem Zapfenrad 50 und dem Gehäuse 40 zu verringern. Entsprechende Materialien und/oder Oberflächen- oder Wärmebehandlungen sind ebenfalls bei weiteren Bauteilen möglich, insbesondere den zusammenwirkenden Zapfen 52 und Zahnreihen 60.

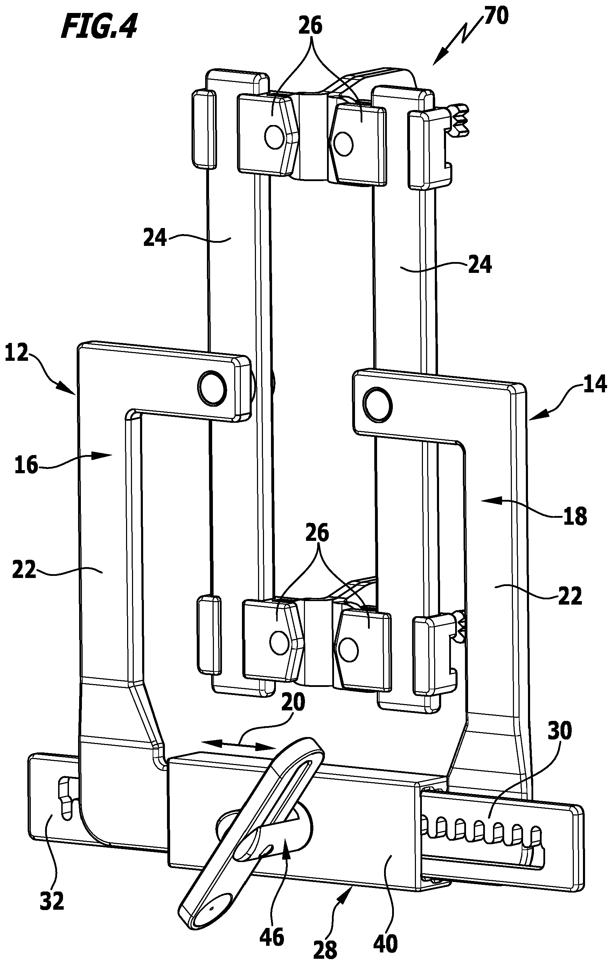

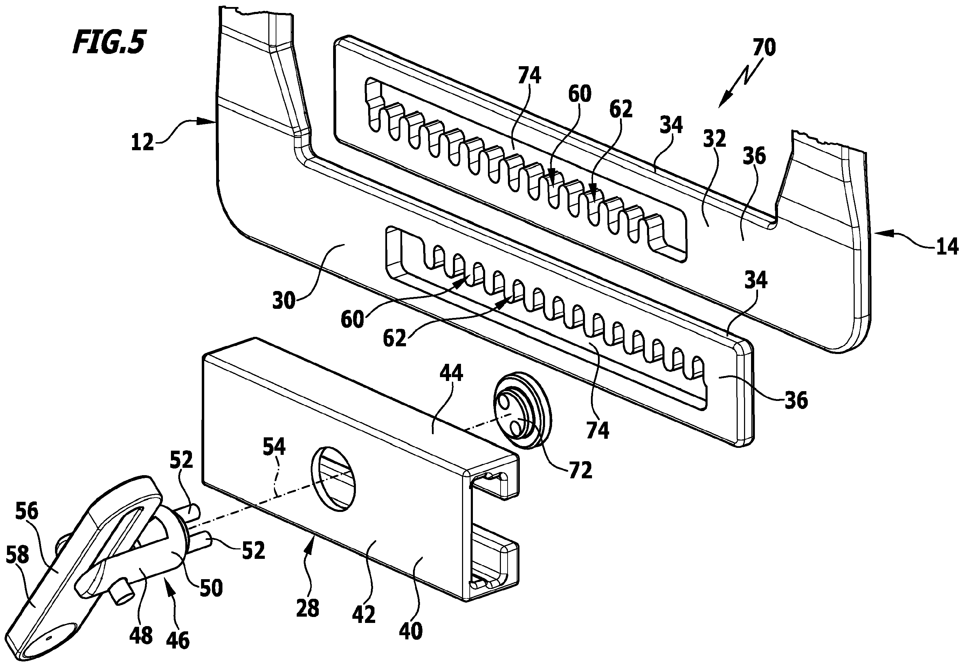

Die Figuren 4 bis 6 zeigen eine zweite, insgesamt mit dem Bezugszeichen 70 belegte vorteilhafte Ausführungsform einer erfindungsgemäßen Retraktionsvorrichtung. Die mit der Retraktionsvorrichtung 10 erzielbaren Vorteile können mit der Retraktionsvorrichtung 70 ebenfalls erzielt werden, so dass diesbezüglich zur Vermeidung von Wiederholungen auf voranstehende Erläuterungen verwiesen werden kann. Für gleiche und gleichwirkende Merkmale und Bauteile der Retraktionsvorrichtungen 10 und 70 werden identische Bezugszeichen benutzt, und es wird auf die wesentlichen Unterschiede eingegangen.

Bei der Retraktionsvorrichtung 70 sind die Haltearme 30, 32 parallel zueinander angeordnet und liegen flächig über ihre Breitseiten 36 aneinander an, so dass sie sich gegenseitig beim Verschieben führen können. Außerdem werden beide Haltearme 30, 32 vom Gehäuse 40 geführt. Das Gehäuse 40 ist bei der Retraktionsvorrichtung 70 in Umfangsrichtung der Haltearme 30, 32 nicht in sich geschlossen. Einer geschlossenen Breitseite 42 gegenüberliegend ist die Breitseite des Gehäuses 40 geöffnet, so dass das Gehäuse 40 im Querschnitt eine C-förmige Gestalt aufweist. Dies dient dazu, das Antriebselement 48 mit einem Fixierelement 72 von der der geschlossenen Breitseite 42 gegenüberliegenden Seite am Gehäuse 40 zu fixieren.

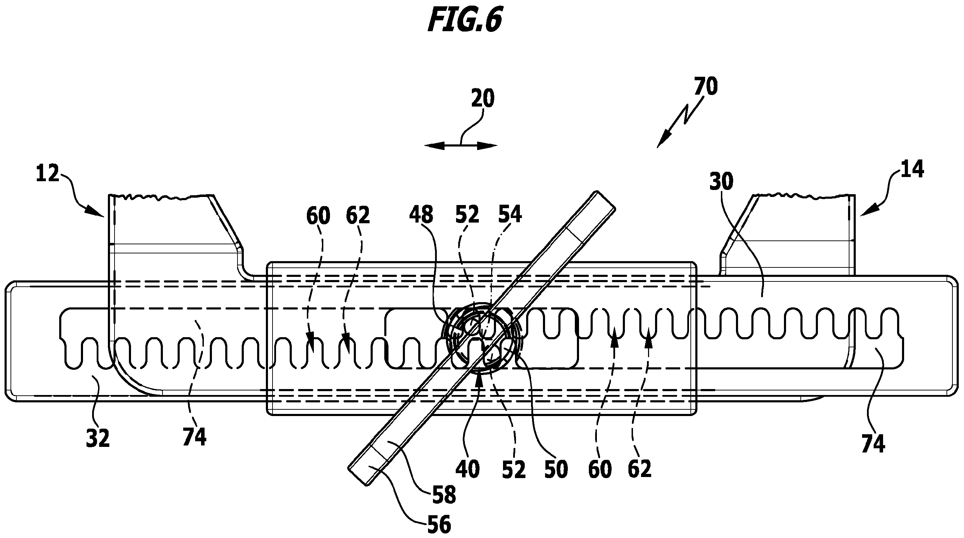

Die Haltearme 30, 32 weisen sich in Längsrichtung (entsprechend der Retraktionsrichtung 20) erstreckende Langlöcher 74 auf. Die Zahnreihen 60 sind an Rändern der Langlöcher 74 angeordnet, wobei die Zähne der Zahnreihen 60 als Vorsprünge der Ränder der Langlöcher 74 gebildet sind. Jede Zahnreihe erstreckt sich im Wesentlichen über einen Längsrand eines Langlochs 74. Die Zähne beider Zahnreihen 60 sind einander zugewandt, so dass das Zapfenrad 50 gleichzeitig mit den Zahnreihen 60 beider Haltearme 30, 32 kämmen kann (Figur 6). Die Zahnreihe 60 beim Haltearm 30 weist in eine von den Rückhalteelementen 26 weg weisende Richtung, und die Zahnreihe 60 beim Haltearm 32 weist in Richtung der Rückhalteelemente 26.

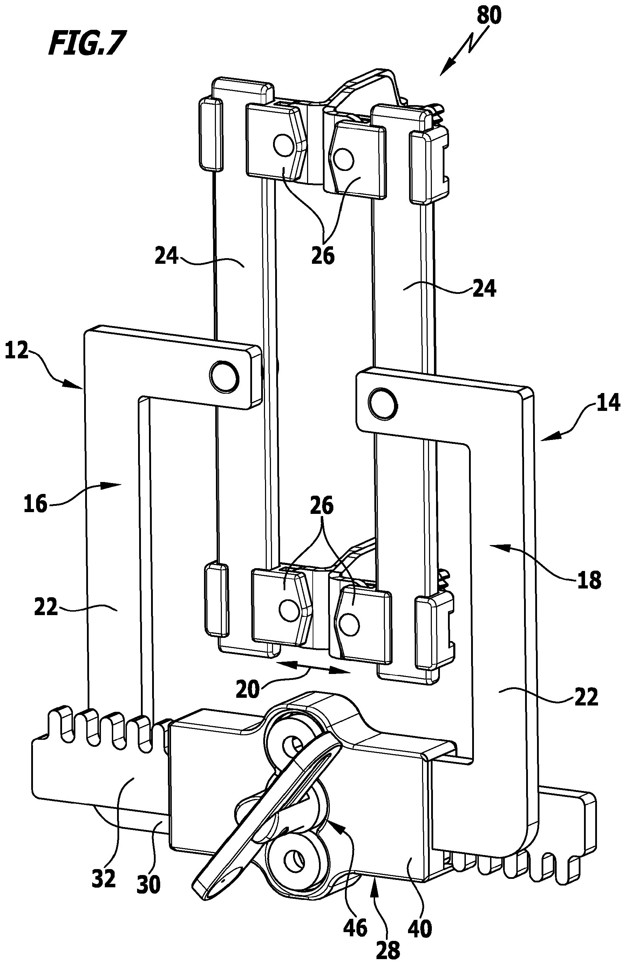

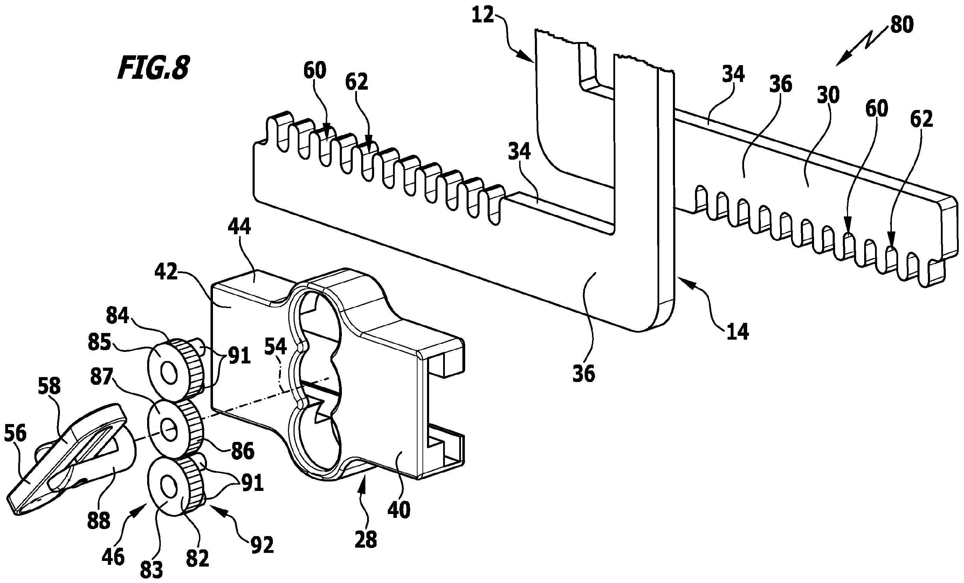

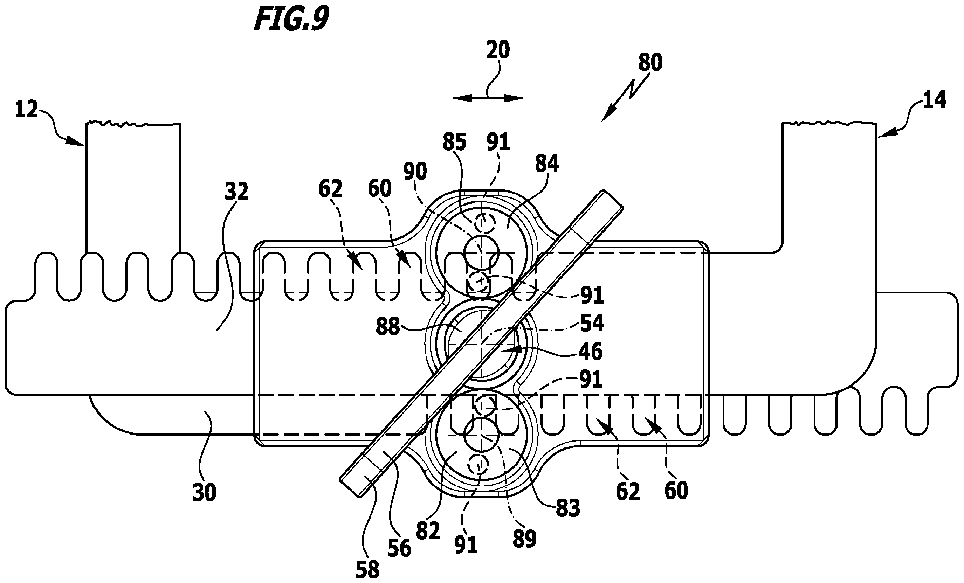

Die Figuren 7 bis 9 zeigen eine insgesamt mit dem Bezugszeichen 80 belegte dritte vorteilhafte Ausführungsform einer erfindungsgemäßen Retraktionsvorrichtung. Die mit der Retraktionsvorrichtung 10 erzielbaren Vorteile können mit der Retraktionsvorrichtung 80 ebenfalls erzielt werden, so dass diesbezüglich zur Vermeidung von Wiederholungen auf vorstehende Erläuterungen verwiesen wird. Für gleiche und gleichwirkende Merkmale und Bauteile der Retraktionsvorrichtungen 10 und 80 werden identische Bezugszeichen benutzt, und es werden die wesentlichen Unterschiede beschrieben.

Bei der Retraktionsvorrichtung 80 liegen die Haltearme 30, 32, ebenso wie bei der Retraktionsvorrichtung 70, über Breitseiten 36 flächig aneinander an. Die Zahnreihen 60 sind an Schmalseiten 34 angeordnet, und zwar in voneinander weg weisenden Richtungen. Beim Haltearm 30 weist die Zahnreihe 60 weg von den Rückhalteelementen 26, und beim Haltearm 32 weist die Zahnreihe 60 in Richtung auf die Rückhalteelemente 26. Die Zahnreihen 60 erstrecken sich vom jeweiligen freien Ende eines Haltearmes 30, 32 über ungefähr 60 % von dessen Länge. Die Zahnreihen 60 sind gebildet, indem an den Haltearmen 30, 32 an den Schmalseiten 34 Ausnehmungen gebildet sind, so dass die nicht ausgesparten Bereiche Zähne der Zahnreihen 60 bilden.

Das Gehäuse 40 ist ebenso wie das Gehäuse 40 der Retraktionsvorrichtung 70 in Umfangsrichtung der Haltearme 30, 32 nicht in sich geschlossen, sondern es weist eine im Querschnitt im Wesentlichen C-förmige Gestalt auf. Mittig umfasst das Gehäuse 40 an den Schmalseiten 44 zwei konvexe Ausbauchungen, so dass innerhalb des Gehäuses 40 ein Aufnahmeraum geschaffen ist für zwei Antriebselemente 82 und 84 der Antriebseinrichtung 46. Die Antriebselemente 82, 84 sind ausgestaltet als Zahnräder 83 bzw. 85. Zwischen den Zahnrädern 83, 85 ist ein weiteres Antriebselement 86 in Gestalt eines Zahnrades 87 angeordnet, welches gleichzeitig mit beiden Zahnrädern 83 und 85 kämmt und drehfest mit einem Antriebskörper 88 der Antriebseinrichtung 46 verbunden ist. Der Antriebskörper 88 ersetzt das Zapfenrad 50 der Retraktionsvorrichtung 10 bei der Retraktionsvorrichtung 80, und an ihm ist die Kurbel 58 festgelegt. Der Antriebskörper 88 und damit das Zahnrad 87 sind um die Drehachse 54 senkrecht zur Spreizebene drehbar am Gehäuse 40 gelagert. Bei einer Drehung des Zahnrades 87 werden die Zahnräder 83 und 85 um Drehachsen 89 bzw. 90 senkrecht zur Spreizebene gleichzeitig gedreht. Alle Drehachsen 54, 89, 90 sind in einer gemeinsamen Ebene, nämlich der Mittelebene der Retraktionsvorrichtung 80, angeordnet.

An Stirnflächen der Zahnräder 83, 85 sind jeweils zwei Zapfen 91 angeordnet, die bezüglich der jeweiligen Drehachse 89 bzw. 90 einander diametral gegenüberstehen und in Eingriff mit den Zahnreihen 60 stehen. Beim Drehen der Kurbel 58 wird dadurch jeder Haltearm 30, 32 drehend angetrieben, so dass die Haltearme 30, 32 relativ zueinander und relativ zum Gehäuse 40 längs der Retraktionsrichtung 20 gegengleich verschoben werden können.

Das Vorsehen der Zahnräder 83, 85, 87 erlaubt es, mit der Antriebseinrichtung 46 ein Getriebe 92 auszubilden. Eine Drehzahl (Anzahl der Umdrehungen) der Kurbel 58 kann durch das Getriebe 92 über- oder untersetzt werden, um die Drehzahl der Zahnräder 83, 85 den Anforderungen entsprechend anzupassen. Abhängig von der Über- oder Untersetzung können die Haltearme 30, 32 unterschiedlich weit relativ zueinander bei jeder Umdrehung der Kurbel 58 verschoben werden.

Es kann vorgesehen sein, dass die Zahnräder 83, 85 und 87 austauschbar sind, um die Über- oder Untersetzung anpassen zu können, wodurch der Retraktionsvorrichtung 80 eine hohe Vielseitigkeit verliehen wird.

Die vorstehende Beschreibung umfasst insbesondere die Offenbarung der anhand nachfolgender Sätze definierten vorteilhaften Ausführungsformen einer erfindungsgemäßen chirurgischen Retraktionsvorrichtung:
1. Chirurgische Retraktionsvorrichtung, insbesondere zum Spreizen eines durchtrennten Sternums, umfassend eine erste Rückhalteeinrichtung (12) und eine zweite Rückhalteeinrichtung (14), die jeweils einen Spreizarm (16, 18) mit mindestens einem daran gehaltenen Rückhalteelement (26) aufweisen, eine Halteeinrichtung (28) zum Halten der Rückhalteeinrichtungen (12, 14) aneinander sowie eine Antriebseinrichtung (46), mit der der Abstand der Spreizarme (16, 18) voneinander längs einer Retraktionsrichtung (20) zum Überführen in eine Spreizstellung veränderbar ist, dadurch gekennzeichnet, dass die Rückhalteeinrichtungen (12, 14) jeweils einen mit dem jeweiligen Spreizarm (16, 18) verbundenen Haltearm (30, 32) aufweisen, wobei beide Haltearme (30, 32) mit der Antriebseinrichtung (46) koppeln und über diese relativ zueinander und relativ zur Halteeinrichtung (28) längs einer Verschieberichtung (20) verschieblich sind.
2. Retraktionsvorrichtung nach Satz 1, dadurch gekennzeichnet, dass die Verschieberichtung (20) parallel zur Retraktionsrichtung (20) ausgerichtet ist.
3. Retraktionsvorrichtung nach Satz 1 oder 2, dadurch gekennzeichnet, dass die Halteeinrichtung (28) und/oder die Antriebseinrichtung (46), bezogen auf die Retraktionsrichtung (20), in der Mitte oder im Wesentlichen in der Mitte zwischen den Spreizarmen (16, 18) angeordnet ist.
4. Retraktionsvorrichtung nach einem der voranstehenden Sätze, dadurch gekennzeichnet, dass die Halteeinrichtung (28) und/oder die Antriebseinrichtung (46) in oder im Wesentlichen in einer von den Spreizarmen (16, 18) definierten Spreizebene angeordnet ist.
5. Retraktionsvorrichtung nach einem der voranstehenden Sätze, dadurch gekennzeichnet, dass die Haltearme (30, 32) bei Betätigung der Antriebseinrichtung (46) um dieselbe Wegstrecke relativ zur Halteeinrichtung (28) verschiebbar sind.
6. Retraktionsvorrichtung nach einem der voranstehenden Sätze, dadurch gekennzeichnet, dass die Haltearme (30, 32) schienenförmig ausgestaltet sind.
7. Retraktionsvorrichtung nach einem der voranstehenden Sätze, dadurch gekennzeichnet, dass die Halteeinrichtung (28) ein Gehäuse (40) umfasst oder ausbildet, das die Haltearme (30, 32) zumindest teilweise umgreift.
8. Retraktionsvorrichtung nach Satz 7, dadurch gekennzeichnet, dass das Gehäuse (40) die Haltearme (30, 32) vollständig oder im Wesentlichen vollständig umgreift.
9. Retraktionsvorrichtung nach Satz 7 oder 8, dadurch gekennzeichnet, dass das Gehäuse (40) als Flachgehäuse ausgestaltet ist.
10. Retraktionsvorrichtung nach einem der Sätze 7 bis 9, dadurch gekennzeichnet, dass die Haltearme (30, 32) von einander gegenüberliegenden Seiten in das Gehäuse (40) eingreifen und/oder dieses durchgreifen.
11. Retraktionsvorrichtung nach einem der voranstehenden Sätze, dadurch gekennzeichnet, dass die Halteeinrichtung (28) eine Führung für zumindest einen der Haltearme (30, 32) bildet.
12. Retraktionsvorrichtung nach einem der voranstehenden Sätze, dadurch gekennzeichnet, dass die Haltearme (30, 32) aneinander anliegen und sich beim Verschieben relativ zueinander gegenseitig führen.
13. Retraktionsvorrichtung nach einem der voranstehenden Sätze, dadurch gekennzeichnet, dass die Haltearme (30, 32) Breitseiten (36) und Schmalseiten (34) aufweisen und dass die Haltearme (30, 32) über die jeweiligen Breitseiten (36) flächig aneinander anliegen.
14. Retraktionsvorrichtung nach einem der voranstehenden Sätze, dadurch gekennzeichnet, dass die Antriebseinrichtung (46) an der Halteeinrichtung (28) angeordnet ist oder von dieser umfasst wird.
15. Retraktionsvorrichtung nach einem der voranstehenden Sätze, dadurch gekennzeichnet, dass die Antriebseinrichtung (46) mindestens ein Antriebselement (48) umfasst und dass die Haltearme (30, 32) jeweils eine sich in Verschieberichtung erstreckende Antriebsbahn (62) umfassen, wobei das mindestens eine Antriebselement (48) mit der jeweiligen Antriebsbahn (62) zusammenwirkt.
16. Retraktionsvorrichtung nach Satz 15, dadurch gekennzeichnet, dass die Antriebsbahn (62) als am jeweiligen Haltearm (30, 32) angeordnete Zahnreihe (60) ausgestaltet ist und dass das mindestens eine Antriebselement (48) als Zapfenrad (50) oder Zahnrad (83, 85, 87) ausgestaltet ist, das mit der Zahnreihe (60) kämmt.
17. Retraktionsvorrichtung nach Satz 15 oder 16, dadurch gekennzeichnet, dass das mindestens eine Antriebselement (48) an der Halteeinrichtung (28) drehbar gelagert ist um eine Drehachse (54) senkrecht zur Verschieberichtung (20).
18. Retraktionsvorrichtung nach einem der Sätze 15 bis 17, dadurch gekennzeichnet, dass die Antriebseinrichtung (46) zum Einwirken auf das mindestens eine Antriebselement (48) mindestens ein handbetätigbares Betätigungselement (56) umfasst, vorzugsweise in Gestalt einer Kurbel (58) oder eines Drehgriffes.
19. Retraktionsvorrichtung nach Satz 18, dadurch gekennzeichnet, dass das Betätigungselement (56) entfernbar ist.
20. Retraktionsvorrichtung nach einem der voranstehenden Sätze, dadurch gekennzeichnet, dass die Retraktionsvorrichtung (70) eine Fixiereinrichtung umfasst, mit der die Rückhalteeinrichtungen (12, 14) in einer Spreizstellung fixierbar sind.
21. Retraktionsvorrichtung nach einem der Sätze 15 bis 19, dadurch gekennzeichnet, dass mindestens ein Haltearm (30, 32) ein längs der Verschieberichtung ausgerichtetes Langloch (74) umfasst, an dessen Rand die Antriebsbahn (62) angeordnet ist.
22. Retraktionsvorrichtung nach einem der Sätze 15 bis 21, dadurch gekennzeichnet, dass die Antriebsbahnen (62) an einander abgewandten Seiten der Haltearme (30, 32) angeordnet sind.
23. Retraktionsvorrichtung nach einem der Sätze 15 bis 22, dadurch gekennzeichnet, dass die Haltearme (30, 32) Breitseiten (36) und Schmalseiten (34) aufweisen und dass die Antriebsbahnen (62) an den Schmalseiten (34) angeordnet sind.
24. Retraktionsvorrichtung nach einem der Sätze 15 bis 23, dadurch gekennzeichnet, dass die Antriebseinrichtung (46) ein Antriebselement (48) umfasst, das mit den Antriebsbahnen (62) beider Haltearme (30, 32) zusammenwirkt.
25. Retraktionsvorrichtung nach Satz 24, dadurch gekennzeichnet, dass die Antriebsbahnen (62) der Haltearme (30, 32) einander zugewandt sind und dass das Antriebselement (48) in einen Raum zwischen den Antriebsbahnen (62) eingreift.
26. Retraktionsvorrichtung nach einem der Sätze 15 bis 23, dadurch gekennzeichnet, dass die Antriebseinrichtung (46) zwei Antriebselemente (82, 84) umfasst, wobei jedes Antriebselement (82, 84) mit einer Antriebsbahn (62) eines Haltearmes (30, 32) zusammenwirkt.
27. Retraktionsvorrichtung nach Satz 26, dadurch gekennzeichnet, dass die Antriebselemente (48) gemeinsam antreibbar sind, bevorzugt dass die Antriebseinrichtung (46) ein mit dem Betätigungselement (56) gekoppeltes Antriebselement (86) umfasst, das mit den beiden Antriebselementen (82, 84) kämmt.
28. Retraktionsvorrichtung nach einem der voranstehenden Sätze, dadurch gekennzeichnet, dass die Antriebseinrichtung (46) ein Getriebe (92) umfasst oder ausbildet, mit der eine Drehzahl des als Drehkörper, insbesondere als Zahnrad (83, 85) oder Zapfenrad (50), ausgestalteten Antriebselementes (82, 84) veränderbar ist.
29. Retraktionsvorrichtung nach Satz 28, dadurch gekennzeichnet, dass das Getriebe (92) in einem von der Halteeinrichtung (28) umfassten oder gebildeten Gehäuse (40) aufgenommen ist.
30. Retraktionsvorrichtung nach einem der voranstehenden Sätze, dadurch gekennzeichnet, dass zumindest einer der Haltearme (30) eine Führung an der jeweils anderen Rückhalteeinrichtung (14) durchgreift.
31. Retraktionsvorrichtung nach einem der voranstehenden Sätze, dadurch gekennzeichnet, dass jeder Haltearm (30, 32) einstückig mit dem jeweiligen Spreizarm (16, 18) verbunden ist.

## Patentansprüche

1. Chirurgische Retraktionsvorrichtung, insbesondere zum Spreizen eines durchtrennten Sternums, umfassend eine erste Rückhalteeinrichtung (12) und eine zweite Rückhalteeinrichtung (14), die jeweils einen Spreizarm (16, 18) mit mindestens einem daran gehaltenen Rückhalteelement (26) aufweisen, eine Halteeinrichtung (28) zum Halten der Rückhalteeinrichtungen (12, 14) aneinander sowie eine Antriebseinrichtung (46), mit der der Abstand der Spreizarme (16, 18) voneinander längs einer Retraktionsrichtung (20) zum Überführen in eine Spreizstellung veränderbar ist, **dadurch gekennzeichnet, dass** die Rückhalteeinrichtungen (12, 14) jeweils einen mit dem jeweiligen Spreizarm (16, 18) verbundenen Haltearm (30, 32) aufweisen, wobei beide Haltearme (30, 32) mit der Antriebseinrichtung (46) koppeln und über diese relativ zueinander und relativ zur Halteeinrichtung (28) längs einer Verschieberichtung (20) verschieblich sind.

2. Retraktionsvorrichtung nach Anspruch 1, **dadurch gekennzeichnet, dass** die Verschieberichtung (20) parallel zur Retraktionsrichtung (20) ausgerichtet ist.

3. Retraktionsvorrichtung nach Anspruch 1 oder 2, **dadurch gekennzeichnet, dass** die Halteeinrichtung (28) und/oder die Antriebseinrichtung (46) in oder im Wesentlichen in einer von den Spreizarmen (16, 18) definierten Spreizebene angeordnet ist.

4. Retraktionsvorrichtung nach einem der voranstehenden Ansprüche, **dadurch gekennzeichnet, dass** die Haltearme (30, 32) bei Betätigung der Antriebseinrichtung (46) um dieselbe Wegstrecke relativ zur Halteeinrichtung (28) verschiebbar sind.

5. Retraktionsvorrichtung nach einem der voranstehenden Ansprüche, **dadurch gekennzeichnet, dass** die Halteeinrichtung (28) ein Gehäuse (40) umfasst oder ausbildet, das die Haltearme (30, 32) zumindest teilweise umgreift, vorzugsweise dass das Gehäuse (40) die Haltearme (30, 32) vollständig oder im Wesentlichen vollständig umgreift.

6. Retraktionsvorrichtung nach einem der voranstehenden Ansprüche, **dadurch gekennzeichnet, dass** die Haltearme (30, 32) aneinander anliegen und sich beim Verschieben relativ zueinander gegenseitig führen und/oder dass die Haltearme (30, 32) Breitseiten (36) und Schmalseiten (34) aufweisen und dass die Haltearme (30, 32) über die jeweiligen Breitseiten (36) flächig aneinander anliegen.

7. Retraktionsvorrichtung nach einem der voranstehenden Ansprüche, **dadurch gekennzeichnet, dass** die Antriebseinrichtung (46) mindestens ein Antriebselement (48) umfasst und dass die Haltearme (30, 32) jeweils eine sich in Verschieberichtung erstreckende Antriebsbahn (62) umfassen, wobei das mindestens eine Antriebselement (48) mit der jeweiligen Antriebsbahn (62) zusammenwirkt, insbesondere dass das mindestens eine Antriebselement (48) an der Halteeinrichtung (28) drehbar gelagert ist um eine Drehachse (54) senkrecht zur Verschieberichtung (20).

8. Retraktionsvorrichtung nach Anspruch 7, **dadurch gekennzeichnet, dass** die Antriebseinrichtung (46) zum Einwirken auf das mindestens eine Antriebselement (48) mindestens ein handbetätigbares Betätigungselement (56) umfasst, vorzugsweise in Gestalt einer Kurbel (58) oder eines Drehgriffes, wobei das Betätigungselement (56) vorzugsweise entfernbar ist.

9. Retraktionsvorrichtung nach einem der voranstehenden Ansprüche, **dadurch gekennzeichnet, dass** die Retraktionsvorrichtung (70) eine Fixiereinrichtung umfasst, mit der die Rückhalteeinrichtungen (12, 14) in einer Spreizstellung fixierbar sind.

10. Retraktionsvorrichtung nach einem der Ansprüche 7 bis 9, **dadurch gekennzeichnet, dass** mindestens ein Haltearm (30, 32) ein längs der Verschieberichtung ausgerichtetes Langloch (74) umfasst, an dessen Rand die Antriebsbahn (62) angeordnet ist.

11. Retraktionsvorrichtung nach einem der Ansprüche 7 bis 10, **dadurch gekennzeichnet, dass** die Antriebsbahnen (62) an einander abgewandten Seiten der Haltearme (30, 32) angeordnet sind.

12. Retraktionsvorrichtung nach einem der Ansprüche 7 bis 11, **dadurch gekennzeichnet, dass** die Haltearme (30, 32) Breitseiten (36) und Schmalseiten (34) aufweisen und dass die Antriebsbahnen (62) an den Schmalseiten (34) angeordnet sind.

13. Retraktionsvorrichtung nach einem der Ansprüche 7 bis 12, **dadurch gekennzeichnet, dass** die Antriebseinrichtung (46) ein Antriebselement (48) umfasst, das mit den Antriebsbahnen (62) beider Haltearme (30, 32) zusammenwirkt, vorzugsweise dass die Antriebsbahnen (62) der Haltearme (30, 32) einander zugewandt sind und das Antriebselement (48) in einen Raum zwischen den Antriebsbahnen (62) eingreift.

14. Retraktionsvorrichtung nach einem der Ansprüche 7 bis 13, **dadurch gekennzeichnet, dass** die Antriebseinrichtung (46) zwei Antriebselemente (82, 84) umfasst, wobei jedes Antriebselement (82, 84) mit einer Antriebsbahn (62) eines Haltearmes (30, 32) zusammenwirkt, insbesondere dass die Antriebselemente (48) gemeinsam antreibbar sind, bevorzugt dass die Antriebseinrichtung (46) ein mit dem Betätigungselement (56) gekoppeltes Antriebselement (86) umfasst, das mit den beiden Antriebselementen (82, 84) kämmt.

15. Retraktionsvorrichtung nach einem der voranstehenden Ansprüche, **dadurch gekennzeichnet, dass** die Antriebseinrichtung (46) ein Getriebe (92) umfasst oder ausbildet, mit der eine Drehzahl des als Drehkörper, insbesondere als Zahnrad (83, 85) oder Zapfenrad (50), ausgestalteten Antriebselementes (82, 84) veränderbar ist, insbesondere dass das Getriebe (92) in einem von der Halteeinrichtung (28) umfassten oder gebildeten Gehäuse (40) aufgenommen ist.
